(19) Europäisches Patentamt
European Patent Office
Office européen des brevets

(11) Numéro de publication: **0 251 832**
**A1**

(12) # DEMANDE DE BREVET EUROPEEN

(21) Numéro de dépôt: **87401136.4**

(22) Date de dépôt: **21.05.87**

(51) Int. Cl.⁴: **C 07 D 211/14**
C 07 D 211/46,
C 07 D 295/12, A 61 K 31/445

(30) Priorité: **30.05.86 US 868443**

(43) Date de publication de la demande:
**07.01.88 Bulletin 88/01**

(84) Etats contractants désignés:
**AT BE CH DE ES FR GB GR IT LI LU NL SE**

(71) Demandeur: **SOCIETE DE RECHERCHES INDUSTRIELLES S.O.R.I.**
**38, avenue Hoche**
**F-75008 Paris (FR)**

(72) Inventeur: **Majoie, Bernard**
**71, rue Montchapet**
**F-21000 Dijon (FR)**

**Bellamy, François**
**rue Basse-Cidex 11**
**F-21910 Sauion-La-Rue (FR)**

**Dodey, Pierre**
**21 rue des Buttes**
**F-21000 Dijon (FR)**

**Robin, Jacques**
**19 rue de la Corvée**
**F-21000 Dijon (FR)**

(74) Mandataire: **Combe, André et al**
**CABINET BEAU DE LOMENIE 55, rue d'Amsterdam**
**F-75008 Paris (FR)**

(54) **Procédé de préparation de dérivés de benzoylphénylpipéridine.**

(57) La présente invention concerne un procédé de préparation d'un produit de formule :

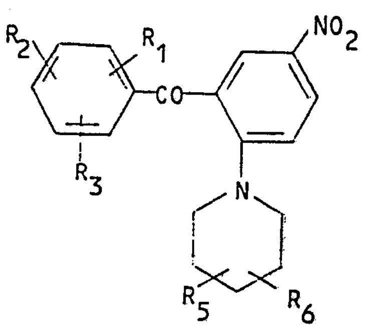

$(I_o)$

dans laquelle $R_1$, $R_2$ et $R_3$ représentent un hydrogène, un halogène, un alkyle inférieur ou un alkoxy inférieur ; - $R_5$ et $R_6$ représentent un hydrogène, un hydroxyle, un alkyle inferieur, un groupe phényle ou un groupe benzyle, par réaction d'une 2-halogéno-5-nitro-benzophénone de formule (II)

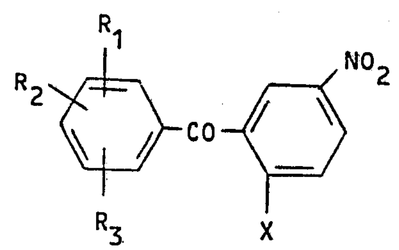

(II)

(III) dans laquelle X est un atome d'halogène avec une pipéridine de formule

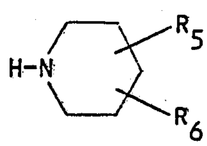

(III)

ledit procédé étant caractérisé en ce que ladite réaction est réalisée dans un milieu contenant au moins 20 % en volume d'eau et pouvant contenir jusqu'à 80 % en volume d'au moins un hydrocarbure aromatique, de préférence le toluène.

EP 0 251 832 A1

## Description

Procédé de préparation de dérivés de benzoylphénylpipéridine.

La présente invention vise à un procédé de préparation de dérivés de benzoylphénylpipéridine de formule

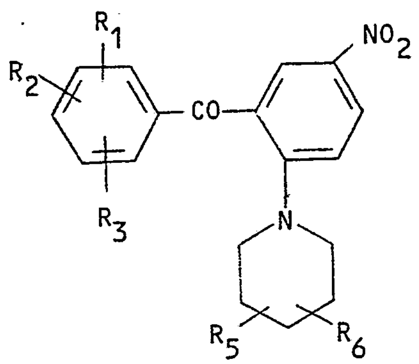

(I$_o$)

dans laquelle
- $R_1$, $R_2$ et $R_3$,identiques ou différents, représentent chacun un atome d'hydrogène, un atome d'halogène, un alkyle inférieur ou un alkoxy inférieur,
- $R_5$ et $R_6$, identiques ou différents, représentent chacun un atome d'hydrogène, un groupe hydroxyle, un alkyle inférieur, un groupe phényle ou un groupe benzyle.

Dans cette définition, on appelle "alkyle inférieur" et "alkoxy inférieur" des radicaux ayant 1 à 4 atomes de carbone.

Le procédé selon la présente invention consiste à faire réagir une 2-halogéno-5-nitrobenzophénone de formule (II)

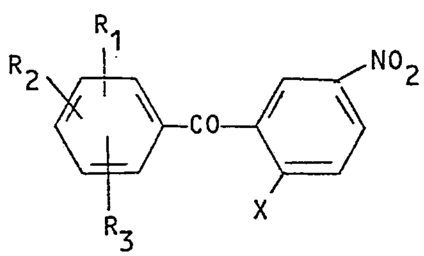

(II)

X étant un atome d'halogène, de préférence Cl ou F, et $R_1$, $R_2$, $R_3$ étant définis comme indiqué ci-dessus, avec une pipéridine éventuellement substituée de formule (III)

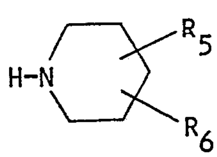

(III)

où $R_5$ et $R_6$ sont tels que définis ci-dessus, ladite réaction étant caractérisée en ce qu'elle est réalisée dans un milieu aqueux contenant au moins 20 % en volume d'eau et qui peut contenir jusqu'à 80 % en volume d'au moins un hydrocarbure aromatique, de préférence le toluène.

Les produits de formule (I$_o$) sont utilisables notamment comme produits intermédiaires en vue de la préparation de produits pharmaceutiquement actifs dans lesquels le groupe $NO_2$ desdits produits a été transformé, par réduction, en groupe $NH_2$. Lesdits produits actifs ont été précédemment décrits.

La préparation des produits de formule (I$_o$) par réaction d'un produit de formule (II) sur un produit de formule (III) a également été décrite ; mais,dans l'art antérieur, cette réaction a toujours été réalisée dans un milieu organique anhydre.

La caractéristique de la présente invention est donc que ladite réaction est effectuée dans un milieu qui contient au moins 20 % en volume d'eau et qui peut même être constitué d'eau pure.

Pour l'homme de l'art, il n'était pas évident que l'on puisse réaliser la réaction d'un produit de formule (II) sur un produit de formule (III) dans un milieu aqueux et cela d'autant plus que les réactifs utilisés ne sont pas solubles dans ledit milieu aqueux,si bien que la réaction selon la présente invention est une réaction hétérogène.

Or, il se trouve que,malgré le caractère hétérogène de ladite réaction, le procédé selon l'invention présente

des avantages notables inattendus, à savoir :

- une amélioration du rendement,
- une possibilité de gain d'énergie,
- une beaucoup plus grande facilité pour isoler le produit obtenu.

Les exemples non limitatifs suivants illustrent l'invention.

Exemple 1

Préparation de la 2-(4-méthyl-1-pipéridinyl)-5-nitro-4'-chlorobenzophénone dans un milieu constitué d'eau pure.

Un mélange de 0,024 mole (0,96 g) de NaOH, 25 ml d'eau, 0,02 mole (5,92 g) de 2,4'-dichloro-5-nitrobenzophénone et 0,022 mole (2,6 ml) de 4-méthylpipéridine est chauffé à 80°C pendant 3 h 30 min. Après refroidissement à température ambiante, le précipité obtenu est filtré, lavé à l'eau et séché. On a obtenu 6,74 g (rendement : 94 %) du produit visé qui a un point de fusion de 138°C.

Exemple 2

En opérant de la même façon que dans l'exemple 1, mais en utilisant comme réactif diverses pipéridines, on a pu obtenir les produits suivants :

a) avec la 2-méthylpipéridine, on a obtenu la 2-(2-méthyl-1-pipéridinyl)-5-nitro-4'-chlorobenzophénone avec un rendement de 91 % en 24 h, le produit obtenu ayant un point de fusion de 140°C,

b) avec la 3-méthylpipéridine, on a obtenu la 2-(3-méthyl-1-pipéridinyl)-5-nitro-4'-chlorobenzophénone avec un rendement de 97 % en 3 h, le produit obtenu ayant un point de fusion de 112°C,

c) avec la 3,5-diméthylpipéridine, on a obtenu la 2-(3,5-diméthyl-1-pipéridinyl)-5-nitro-4'-chlorobenzophénone avec un rendement de 95,8 % en 2 h, le produit ayant, après recristallisation du toluène, un point de fusion de 171°C.

Exemple 3

En opérant comme dans l'exemple 1, mais en utilisant, avec la 4-méthylpipéridine, divers dérivés de la 2-chloro-5-nitrobenzophénone, on a obtenu les produits suivants :

a) la 2-(4-méthyl-1-pipéridinyl)-5-nitro-3'-chlorobenzophénone avec un rendement de 93,4 % en 2 h, ledit produit ayant un point de fusion de 86°C,

b) la 2-(4-méthyl-1-pipéridinyl)-5-nitro-4'-méthylbenzophénone avec un rendement de 81 % en 1 h 30 min, ledit produit ayant, après recristallisation de l'éther isopropylique, un point de fusion de 110°C,

c) la 2-(4-méthyl-1-pipéridinyl)-5-nitro-4'-méthoxybenzophénone avec un rendement de 88,4 % en 1 h, ledit produit ayant, après recristallisation de l'éther isopropylique, un point de fusion de 117°C,

d) la 2-(4-méthyl-1-pipéridinyl)-5-nitro-3',4'-dichlorobenzophénone avec un rendement de 97 % en 9 h, ledit produit ayant un point de fusion de 114°C,

e) la 2-(4-méthyl-1-pipéridinyl)-5-nitro-2',4'-diméthylbenzophénone avec un rendement de 97,5 % en 4 h, ledit produit ayant un point de fusion de 106°C,

f) la 2-(4-méthyl-1-pipéridinyl)-5-nitro-2'-chlorobenzophénone avec un rendement de 95 % en 3 h, ledit produit ayant, après lavage à l'éther isopropylique, un point de fusion de 84°C.

Exemple 4

Préparation de la 2-(4-méthyl-1-pipéridinyl)-5-nitro-4'-chlorobenzophénone dans un milieu constitué d'un mélange d'eau et de toluène.

Un mélange de 0,024 mole (0,96 g) de NaOH, 5 ml d'eau, 15 ml de toluène, 0,02 mole (5,92 g) de 2,4'-dichloro-5-nitrobenzophénone et 0,022 mole (2,6 ml) de 4-méthylpipéridine est chauffé à 80°C pendant 1 h. Après refroidissement à température ambiante, le précipité obtenu est filtré, lavé à l'eau et séché. On a obtenu 6,51 g (rendement 90,8 %) du produit visé, ledit produit ayant un point de fusion de 138°C.

Exemple 5

En opérant comme dans l'exemple 4, mais en faisant réagir sur la 2,4'-dichloro-5-nitrobenzophénone divers dérivés de la pipéridine, on a obtenu les produits suivants :

a) la 2-(3-méthyl-1-pipéridinyl)-5-nitro-4'-chlorobenzophénone avec un rendement de 94,5 % en 1 h 15 min, le produit ayant un point de fusion de 111°C,

b) la 2-(2-méthyl-1-pipéridinyl)-5-nitro-4'-chlorobenzophénone avec un rendement de 70 % en 5 h, le produit ayant un point de fusion de 140°C,

c) la 2-(3,5-diméthyl-1-pipéridinyl)-5-nitro-4'-chlorobenzophénone avec un rendement de 96,2 % en 3 h, le produit ayant un point de fusion de 171°C après recristallisation dans le toluène.

Exemple 6

En opérant comme dans l'exemple 4, mais en faisant réagir, sur la 4-méthylpipéridine, divers dérivés de la 2-chloro-5-nitrobenzophénone, on a obtenu les produits suivants :

a) la 2-(4-méthyl-1-pipéridinyl)-5-nitro-3'-chlorobenzophénone avec un rendement de 83,6 % en 1 h 30

min, le produit obtenu ayant un point de fusion de 88° C,

b) la 2-(4-méthyl-1-pipéridinyl)-5-nitro-4′-méthylbenzophénone avec un rendement de 97 % en 2 h, le produit obtenu ayant un point de fusion de 110° C,

c) la 2-(4-méthyl-1-pipéridinyl)-5-nitro-4′-méthoxybenzophénone avec un rendement de 68,4 % en 1 h, le produit obtenu ayant, après recristallisation de l'éther isopropylique, un point de fusion de 120° C,

d) la 2-(4-méthyl-1-pipéridinyl)-5-nitro-3′,4′-dichlorobenzophénone avec un rendement de 98,3 % en 3 h, le produit obtenu ayant un point de fusion de 114° C,

e) la 2-(4-méthyl-1-pipéridinyl)-5-nitro-2′,4′-diméthylbenzophénone avec un rendement de 88 % en 2 h, le produit obtenu ayant un point de fusion de 107° C,

f) la 2-(4-méthyl-1-pipéridinyl)-5-nitro-2′-chlorobenzophénone avec un rendement de 95 % en 3 h, le produit obtenu ayant un point de fusion de 84°C après lavage par un mélange pentane-éther isopropylique.

Exemple 6

Cet exemple illustre la préparation de la 2-(4-méthyl-1-pipéridinyl)-5-nitro-4′-chlorobenzophénone, dans un milieu contenant de l'eau et du toluène, en utilisant des quantités notables de réactifs.

Dans un réacteur de 4 l, on introduit sous agitation 1 050 ml de toluène et 1 kg de 2,4′-dichloro-5-nitrobenzophénone. La dissolution du réactif dans le milieu est partielle.

On ajoute ensuite une solution de 162 g de soude dans 500 ml d'eau ; 368 g (440 ml) de 4-méthylpipéridine sont alors introduits en une demi-heure par une ampoule à brome. La réaction est suffisamment exothermique pour que la température du réacteur atteigne 84° C, de telle sorte qu'il n'est pas nécessaire de chauffer ledit mélange au début de la réaction. A la fin de l'addition de la pipéridine, la température est maintenue à 80° C pendant environ 2 h, c'est-à-dire jusqu'à la fin de la réaction. Le produit final cristallise dans le milieu. Environ 16 h après que le milieu a été refroidi jusqu'à température ambiante, le produit cristallisé obtenu est filtré, lavé plusieurs fois à l'eau (jusqu'à obtention d'un pH neutre), puis séché sous vide à 80°C. On obtient 1 140 g (rendement : 94,08 %) du produit visé qui a un point de fusion de 138°C.

On a regroupé dans le tableau I suivant les structures et le point de fusion des exemples précédents ainsi qu'un certain nombre de composés obtenus selon le procédé objet de la présente invention.

TABLEAU I

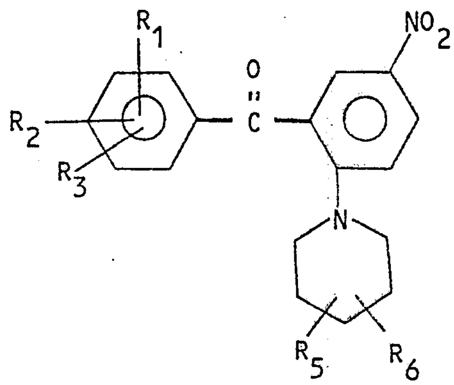

| $R_1$ | $R_2$ | $R_3$ | $R_5$ | $R_6$ | F°C |
|---|---|---|---|---|---|
| 4-Cl | -H | -H | 4-$CH_3$ | H | 138 |
| 2-Cl | -H | H | 4-$CH_3$ | H | 84 |
| 4-Cl | H | H | H | H | 141 |
| 4-Cl | H | H | 3-$CH_3$ | H | 111-112 |
| 4-$CH_3$ | H | H | 4-$CH_3$ | H | 110 |
| 2-$CH_3$ | 4-$CH_3$ | H | 4-$CH_3$ | H | 106-107 |
| 4-Cl | H | H | 3-$CH_3$ | 5-$CH_3$ | 171 |
| 4-Cl | H | H | 2-$CH_3$ | H | 140 |
| 4-$OCH_3$ | H | H | 4-$CH_3$ | H | 117-120 |
| H | H | H | 4-$CH_3$ | H | 81 |
| 3-Cl | 4-Cl | H | 4-$CH_3$ | H | 114 |
| 4-Br | H | H | 4-$CH_3$ | H | 142 |
| 4-Cl | H | H | 4-$CH_2$- 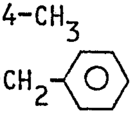 | H | 211 |
| 4-Cl | -H | -H | 4-OH | H | 142 |
| 2-Cl | 4-Cl | H | 4-$CH_3$ | H | 103 |
| 4-Cl | H | H | 4-$CH_3$ | 4-$CH_2CH_3$ | 105 |
| 4-Cl | H | H | 4-$C(CH_3)_3$ | H | 152 |
| 4-Cl | H | H | 4-$C_2H_5$ | H | 129 |
| 4-Cl | H | H | 4n-$C_4H_9$ | H | 105 |
| 4-Cl | H | H | 4- 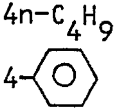 | H | 167 |
| 4-Cl | H | H | 4-$CH_3$ | 4-$CH_3$ | 109,5 |
| 2-Cl | H | H | 4-$C(CH_3)_3$ | H | 171 |
| 4-Cl | H | H | 4-OH | 4- 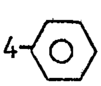 | 203 |
| 3-Cl | H | H | 4-$CH_3$ | H | 86-88 |

**Revendications**

1. Procédé de préparation d'un produit de formule

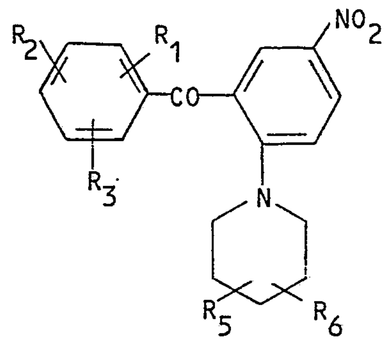

($I_o$)

dans laquelle
- $R_1$, $R_2$, $R_3$, identiques ou différents, représentent chacun un atome d'hydrogène, un atome d'halogène, un alkyle inférieur ou un alkoxy inférieur
- $R_5$ et $R_6$, identiques ou différents, représentent chacun un atome d'hydrogène, un groupe hydroxyle, un alkyle inférieur, un groupe phényle ou un groupe benzyle

par réaction selon une réaction connue d'une 2-halogéno-5-nitrobenzophénone de formule (II)

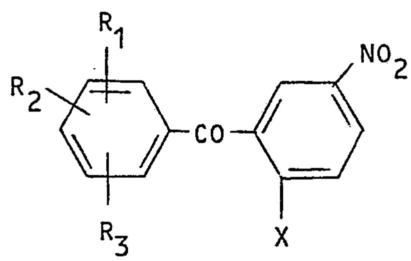

(II)

dans laquelle X est un atome d'halogène, de préférence Cl ou F, et $R_1$, $R_2$, $R_3$ sont tels que définis ci-dessus avec une pipéridine éventuellement substituée de formule (III)

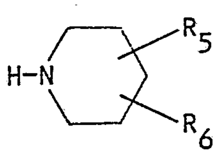

(III)

dans laquelle $R_5$ et $R_6$ sont tels que définis ci-dessus, ledit procédé étant caractérisé en ce que ladite réaction est réalisée dans un milieu contenant au moins 20 % en volume d'eau et pouvant contenir jusqu'à 80 % en volume d'au moins un hydrocarbure aromatique, de préférence le toluène.

2. Procédé de préparation d'un composé de formule (I) selon la revendication 1, caractérisé en ce que l'on fait réagir un composé de formule (II) dans laquelle $R_1$ et $R_2$ représentent l'atome d'hydrogène, $R_3$ représente un groupe 4-Cl et X représente l'atome de chlore, sur un composé de formule (II) dans laquelle $R_5$ représente l'atome d'hydrogène et $R_6$ représente le groupe 4-$CH_3$.

Office européen des brevets

# RAPPORT DE RECHERCHE EUROPEENNE

Numero de la demande

EP 87 40 1136

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X | EP-A-0 069 012 (S.O.R.I.)<br>* Revendications 1,10 *<br>--- | 1,2 | C 07 D 211/14<br>C 07 D 211/46<br>C 07 D 295/12<br>A 61 K 31/445 |
| X | HELVETICA CHIMICA ACTA, vol. 65, Fasc. 7, no. 209, 1982, pages 2118-2133, Bern, CH; W. MÜLLER et al.: "Synthese von 1,2-annelierten 1,4-Benzodiazepinen und 4,1-Benzoxazepinen"<br>* Page 2121, schéma 4, points 34.36; page 2127 *<br>--- | 1-2 | |
| A | CHEMICAL ABSTRACTS, vol. 101, 1984, page 768, résumé no. 230494x, Columbus, Ohio, US; M. KUEHNHOLD et al.: "Problems in the separation of structurally isomeric 2-aminobenzophenones as intermediates in the preparation of annulated 1,4-benzodiazepines"<br>* Abrégé *<br>----- | 1-2 | DOMAINES TECHNIQUES RECHERCHES (Int. Cl.4)<br>C 07 D 211/00<br>C 07 D 295/00 |

Le présent rapport de recherche a été établi pour toutes les revendications

| Lieu de la recherche | Date d'achèvement de la recherche | Examinateur |
|---|---|---|
| LA HAYE | 02-10-1987 | MAISONNEUVE J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82